# EUROPÄISCHE PATENTSCHRIFT

(11) **EP 1 598 127 B1**
(45) Veröffentlichungstag und Bekanntmachung des Hinweises auf die Patenterteilung: **28.05.2008**
(21) Anmeldenummer: 05008598.4
(22) Anmeldetag: 20.04.2005
(51) Int. Cl.: B21D 53/10

(54) **Verfahren zur Herstellung einer Gleitlagerbuchse oder Lagerschale mit variierender Breite**
Method for making a slide bearing bush or bearing shell with varying width
Méthode de production d'un manchon de palier lisse ou coquille de coussinet de largeur variable

(30) Priorität: 19.05.2004 DE 102004024746
(43) Veröffentlichungstag der Anmeldung: 23.11.2005
(73) Patentinhaber: Wieland-Werke AG, 89070 Ulm (DE)
(72) Erfinder: Thumm, Gerhard, 89155 Erbach (DE); Bischof, Ekkehard, 88471 Laupheim (DE)

(56) Entgegenhaltungen:
- DE-A- 2 818 378
- DE-A1- 19 907 571
- US-A- 2 741 023
- US-A- 4 907 626

## Beschreibung

Die Erfindung betrifft ein Verfahren zur Herstellung einer Gleitlagerbuchse oder Lagerschale mit einer variierenden Breite aus einem Flachbandmaterial sowie eine mit dem Verfahren hergestellte gerollte Gleitlagerbuchse oder Lagerschale.

Gleitelemente in Form von Buchsen oder Halbschalen werden oft aus bandförmigem Vormaterial gefertigt. Wegen ihrer rationellen Fertigungsmöglichkeit und der sich daraus ergebenden günstigen Eigenschaften werden gerollte Gleitlagerbuchsen immer häufiger verwendet. Ausgangsmaterial zur Herstellung ist dabei ein Band mit glatter Oberfläche und, in manchen Fällen, eingeprägten Schmiertaschen oder Lochmuster. Die Bänder werden üblicherweise zu Platinen und auf Buchsenkontur geschnitten und anschließend rund gebogen. Jeweilige Ausführungsformen gerollter Gleitelemente können so auf einfache Weise bereits im Bandmaterial an die jeweiligen Anforderungen angepasst werden. Derartige Gleitelemente finden im allgemeinen Maschinenbau, wie beispielsweise bei Bau- und Landmaschinen, und insbesondere als Pleuellagerbuchse im kleinen Pleuelauge oder als Kolbenbuchse im Automotive Bereich Verwendung.

Aus der Offenlegungsschrift DE 199 07 571 A1 ist eine gerollte Gleitlagerbuchse bekannt, die eine über die Umfangsrichtung variierende Buchsenbreite aufweist. Hierzu wird die Buchse aus einem zuvor geformten streifenförmigen Bandabschnitt mit variierender Breite hergestellt. Durch einen weiteren Verfahrensschritt wird vor dem Umformen an den Flankenabschnitten der Formplatine, beispielsweise durch Prägen, eine Fase angebracht. Eine variierende Buchsenbreite soll insbesondere eine Gewichtsersparnis der fest eingepressten Gleitlagerbuchse bewirken und dabei an die geometrischen Randbedingungen eines Pleuels oder Kolbens angepasst sein. Die Gleitlagerbuchse wird nach dem Rollen keiner weiteren spanabhebenden Breitenbearbeitung unterzogen.

Weiterhin ist aus der US 4,907,626 ein aus bandförmigem Material hergestelltes gerolltes Gleitlager bekannt. Das Bandmaterial wird mit Nuten versehen, entlang denen das Band vor dem Umformprozess in Stücke geschnitten wird. Jeder so erhaltene Bandabschnitt wird anschließend zu einer Buchse gerollt.

Die Druckschrift DE 28 18 378 A1 offenbart den nächstliegenden Stand der Technik und beschreibt die Herstellung von Laufbuchsen aus streifenförmigem Rohmaterial, das mit Einkerbungen versehen ist, an denen in einem späteren Herstellungsschritt die Vereinzelung der jeweiligen Buchsen stattfindet. Zuvor wird das streifenförmige Material in aufeinanderfolgenden Schritten in Rohrabschnitte mit kreisförmigem Querschnitt umgeformt und anschließend die Buchsen vom Rohrabschnitt abgetrennt. Die Abtrennung erfolgt an der jeweiligen durch die Einkerbungen zugrunde gelegten gemeinsamen Grenzkerblinien. Mit dieser Methode können je nach Verlauf der Grenzkerblinie auch Buchsen mit variierender Breite hergestellt werden. Der im Verfahren angewandte Trennvorgang beruht in unterschiedlichen Verfahrensschritten auf einer Kombination aus Abscheren bzw. Abschneiden des jeweiligen Materials an der gemeinsamen Grenzkerblinie benachbarter Buchsen.

Ein vergleichbares Verfahren wird auch in der Druckschrift US 2,741,023 offenbart. Ein Unterschied im Verfahrensverlauf beruht darauf, dass ein zur Buchse gerolltes Bandmaterial zwar ebenfalls mit Kerben versehen wird, das Material an diesen Stellen jedoch so ausgedünnt wird, dass es zur Vereinzelung der jeweiligen Lagerbuchse durch die mechanische Einwirkung eines Stempels an diesen Stellen abreißt.

Auf der Grundlage der bisherigen Erkenntnisse liegt der Erfindung die Aufgabe zugrunde, ein Verfahren zur Herstellung von Gleitlagern dahingehend zu verbessern, um, ausgehend von einfachen Bandgeometrien, Gleitlager mit komplexen Formen auf kostengünstige Weise herzustellen.

Die Erfindung wird bezüglich des Verfahrens durch die Merkmale des Anspruchs 1 und bezüglich der Buchse oder Halbschale durch die Merkmale des Anspruchs 9 wiedergegeben. Die weiteren rückbezogenen Ansprüche geben vorteilhafte Aus- und Weiterbildungen der Erfindung wieder.

Die Erfindung schließt die technische Lehre eines Verfahrens zur Herstellung einer Gleitlagerbuchse oder Lagerschale mit einer variierenden Breite aus einem Flachbandmaterial ein, mit folgenden Schritten:
- Einbringen von Nuten im Flachbandmaterial entlang der Buchsen- oder Lagerschalenkontur,
- Rollen oder Biegen des Flachbandmaterials auf Buchsen- oder Lagerschalenform,
- mechanisches Abtrennen der überschüssigen Randbereiche entlang der Nuten,
- Erhalt einer fertigen Gleitlagerbuchse oder Lagerschale, die ihre endgültige Form aufweist.

Die Erfindung geht dabei von der Überlegung aus, Trapez- oder Stufenbuchsen als so genannte Formbuchsen aus Bandmaterial herzustellen. Ausgangspunkt kann dabei ein Bandabschnitt mit rechteckigem Querschnitt sein, der üblicherweise für zylindrische Buchsen verwendet wird. Das Einbringen der Nuten kann im Flachbandmaterial erfolgen. Durch die Nuten wird ein verbleibender Steg aus Restmaterial erzeugt, der eine ausreichend geringe Dicke aufweist, um als Sollbruchstelle für das Vereinzeln zu dienen. Das Flachbandmaterial kann dabei eine Platine sein oder aus einer Platine ausgestanzte rechteckige Abschnitte umfassen. Dabei können beispielsweise auch größere Bandabschnitte zu einem kompakten Rohr gebogen werden, woraus die einzelnen Gleitlager anschließend vereinzelt werden. Das mechanische Abtrennen kann durch bloßes Abbrechen der überschüssigen Randbereiche erfolgen. Eine Nachbearbeitung der Seitenflanken an den gebrochenen Nuten ist entweder entbehrlich oder nur in geringem Maße erforderlich.

Die mit der Erfindung erzielten Vorteile bestehen insbesondere in der Möglichkeit, ausgehend von einfachen Bandgeometrien, Gleitlager mit komplexen Formen auf rationelle und kostengünstige Weise herzustellen. Aus den ursprünglichen einfachen Bandabschnitten lässt sich nahezu jede beliebige Buchsenform erzeugen. Die Grundlage für komplexere Gleitlagerformen ist demnach ein besonders einfach zu handhabender zylindrischer Gleitlagerrohling mit eingebrachten Nuten. Spezifische Werkstoffeigenschaften werden im Fertigungsverfahren entsprechend genutzt. Zudem gibt die Nut an den Seitenflanken der Buchse eine Fase vor, die beim Einbau als Montagehilfe dienen kann. Hierzu können allein durch die Ausbildung der Nuten verschiedene Fasenwinkel vorgegeben werden. So ist beispielsweise eine aufwendige und kostenintensive spanende Bearbeitung von geschrägten oder gestuften Flächen entbehrlich.

Zum Einbringen der Nuten kommen mehrere Verfahrensvarianten in Betracht, die den Gleitelementwerkstoff unterschiedlich beeinflussen. Vorteilhafterweise können die Nuten mittels Prägen als besonders kostengünstiges Verfahren eingebracht werden. Hierbei findet ein lokales Aushärten durch mechanische Kaltumformung statt.

In bevorzugter Ausführungsform können die Nuten mittels Fräsen eingebracht werden. Ein Fräsen ist bei aushärtbaren Legierungen besonders geeignet. Auch das entlang der Nuten verbleibende, vergleichsweise dünne Stegmaterial kann ausgehärtet werden wodurch entsprechende Sollbruchstellen ausgebildet werden, die beim mechanischen Abtrennen bevorzugt aufbrechen.

Alternativ können die Nuten vorteilhafterweise mittels Laserkerben eingebracht werden. Hierdurch kann ein lokales Aushärten entlang der Nuten durch Gefügeumbildung stattfinden. Durch das Aushärten entstehen die für einen Abtrennvorgang benötigten Sollbruchstellen. Vorteilhaft dabei ist, dass bei einer lokalen Gefügeumbildung im Bereich der Nuten das übrige Gleitelementmaterial, insbesondere im Bereich beanspruchter Gleitflächen, in seinen Gleiteigenschaften optimal angepasst werden kann.

In einer weiteren Ausgestaltung können die Nuten alternativ mittels Rollkerben eingebracht werden. Auch bei diesem Verfahren findet ein lokales Aushärten durch mechanische Kaltumformung statt.

Gemäß der Erfindung wird als Werkstoff eine aushärtbare Kupferlegierung verwendet. Vorteilhafterweise finden in diesem Zusammenhang auch Kupferlegierungen auf der Basis von Cu-Sn-Ni und insbesondere CuSn6Ni6 Verwendung.

Gemäß der Erfindung Gemäß des Erfindung wird bei aushärtenden Werkstoffen vor dem mechanischen Abtrennen entlang der Nuten ein Auslagern bei einer Temperatur zwischen 300°C bis 450°C und einer Prozesszeit zwischen 1 h bis 19 h durchgeführt. Hierdurch werden, insbesondere entlang der Nuten, die Sollbruchstellen auf das gewünschte Bruchverhalten beim mechanischen Trennvorgang vorbereitet. Bei der Werkstoffauswahl ist dabei berücksichtigt und erwünscht, dass bei diesem Prozessschritt die Buchse insgesamt einer Aushärtung unterzogen wird.

Vorteilhafterweise wird als Kupferlegierung eine Legierung auf der Basis von Cu-Sn-P, Cu-Zn-Si oder auch Cu-Sn, beispielsweise CuSn8, verwendet.

In weiterer bevorzugter Ausführungsform der Erfindung kann auch ein Werkstoff auf der Basis eines Stahlverbundwerkstoffs oder Mehrstofflagerwerkstoffs verwendet werden.

In bevorzugter Ausgestaltung der Erfindung kann das mechanische Abtrennen entlang der Nuten bei niedriger Temperatur erfolgen. Beispielsweise eignet sich hierzu flüssiger Stickstoff, der das Bruchverhalten beim mechanischen Abtrennen entsprechend positiv beeinflusst.

Vorteilhafterweise können nach dem Abtrennen entlang der Nuten die durch die Nuten vorgegebenen Seitenflächen entgratet werden. Hierzu werden mit geringem Aufwand nur die Rauhigkeiten im aufgebrochenen und ausgesprochen dünnen Stegmaterial geglättet. Die an den Seitenflächen befindlichen Fasen müssen nicht überarbeitet werden. Dies kann in einfacher Weise mit Gleitschleifen erfolgen.

Vorteilhafterweise können Gleitlager beschichtet werden. Hierzu können die bereits bekannten Beschichtungsverfahren vor oder nach dem mechanischen Abtrennen eingesetzt werden.

In bevorzugter Ausgestaltung der Erfindung werden mit dem erfindungsgemäßen Verfahren gerollte Gleitlagerbuchsen oder Halbschalen hergestellt.

Ausführungsbeispiele der Erfindung werden anhand der schematischen Zeichnungen näher erläutert.

Darin zeigen schematisch:
- Fig. 1: zwei einseitig schräge Buchsen vor und nach dem Vereinzeln durch mechanisches Trennen (cracken),
- Fig. 2: eine Ansicht eines zylindrischen Buchsenrohlings mit einer Nut vor dem Abtrennen und eine durch Trennen erhaltene einseitig schräge Buchse,
- Fig. 3: eine Ansicht eines zylindrischen Buchsenrohlings vor dem Abtrennen mit zwei Nuten und eine durch Trennen erhaltene doppelt schräge Buchse,
- Fig. 4: eine Ansicht eines zylindrischen Buchsenrohlings vor dem Abtrennen mit einer Nut und eine durch Trennen erhaltene einseitig gestufte Buchse, und
- Fig. 5: eine Ansicht eines zylindrischen Buchsenrohlings vor dem Abtrennen mit zwei Nuten und eine durch Trennen erhaltene doppelt gestufte Buchse.

Einander entsprechende Teile sind in allen Figuren mit denselben Bezugszeichen versehen.

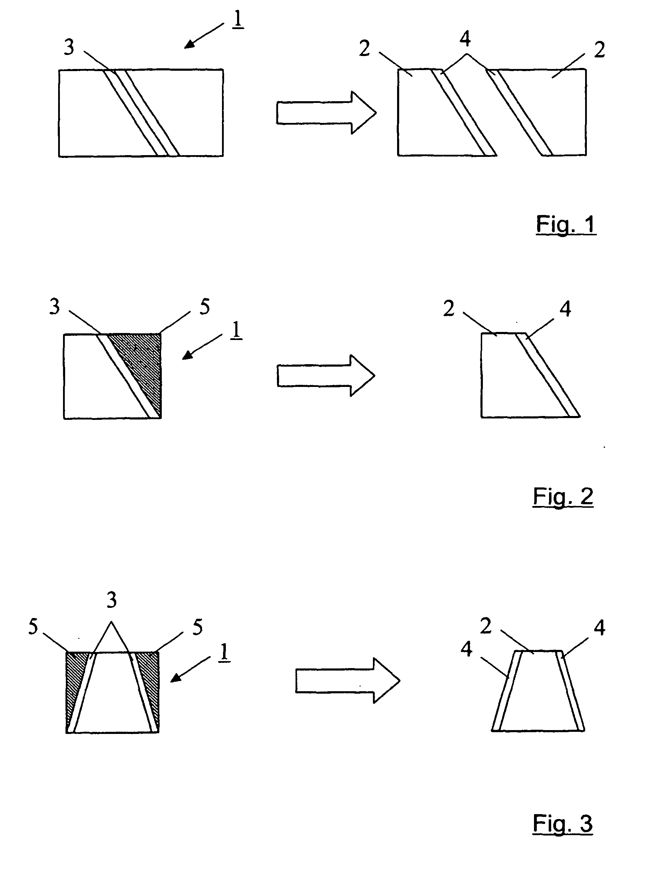

Fig. 1 zeigt schematisch zwei einseitig schräge Buchsen 2 vor und nach dem Vereinzeln durch mechanisches Trennen (cracken). Die Tiefe der Nut 3 im Material ist so ausgeführt, dass diese als Sollbruchstelle für das mechanische Abtrennen entlang dem durch die Nut 3 gebildeten Reststeg dient. Dadurch bildet sich aus den Seitenflächen der Nut 3 im Herstellungsprozess beim Vereinzeln eine Fase 4, die als Montagehilfe für einen Einbau dient. Der Fasenwinkel ist durch den Kerbwinkel beim Einbringen der Nut 3 entsprechend vorgegeben. Bei diesem Ausführungsbeispiel fällt kein Abfall an. Sofern erforderlich, kann die durch ein mechanisches Abtrennen entlang dem Reststeg gebildete Rauhigkeit durch ein Entgraten geglättet werden.

Ausgehend von einem zylindrischen Buchsenrohling 1 mit einer Nut 3 vor dem Abtrennen zeigt Fig. 2 eine Ansicht einer durch Trennen erhaltenen einseitigen schrägen Buchse 2. Der abgetrennte Randbereich 5 kann hierbei als Abfallmaterial wiederverwertet werden.

Fig. 3 zeigt eine Ansicht eines zylindrischen Buchsenrohlings 1 vor dem Abtrennen mit zwei Nuten 3 und eine durch Trennen erhaltene doppelt schräge Buchse 2. Durch die beidseitige Nut 3 wird an beiden Seitenflächen jeweils eine Fase 4 ausgebildet. Das überschüssige Material 5 kann in diesem Falle weitgehend vermieden werden, wenn, analog zu Fig. 1, der zylindrische Buchsenrohling 1 aus einem lang gestreckten rohrförmigen Körper besteht, bei dem eine Vielzahl von Buchsen 2 unmittelbar benachbart angeordnet sind.

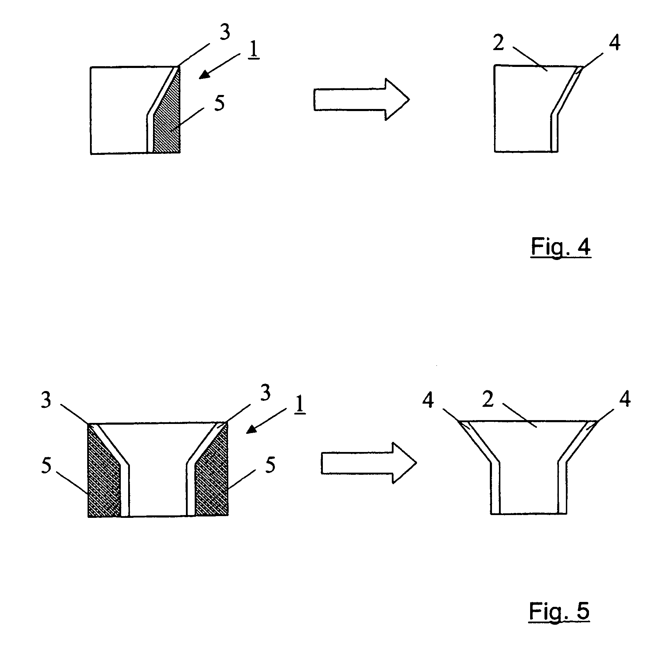

Fig. 4 zeigt eine Ansicht eines zylindrischen Buchsenrohlings 1 vor dem Abtrennen entlang einer Nut 3 und eine durch Trennen erhaltene einseitig gestufte Buchse 2. Die Nut 3 verläuft entsprechend der Kontur der im nachfolgenden Verfahrensschritt erzeugten Buchse 2.

In einem weiteren Ausführungsbeispiel nach Fig. 5 ist eine Ansicht eines zylindrischen Buchsenrohlings 1 vor dem Abtrennen mit zwei Nuten 3 und eine durch Trennen erhaltene doppelt gestufte Buchse 2 dargestellt. An beiden Seitenflächen bilden sich entsprechende Fasen 4 aus.

### Bezugszeichenliste

- 1: zylindrischer Buchsenrohling
- 2: Buchse
- 3: Nut
- 4: Fase
- 5: Randbereich

## Patentansprüche

1. Verfahren zur Herstellung einer Gleitlagerbuchse oder Lagerschale (2) mit einer variierenden Breite aus einem Flachbandmaterial; bestehend aus einer aushärtbaren Kupferlegierung, **gekennzeichnet durch** folgende Schritte:
- Einbringen von Nuten (3) im Flachbandmaterial entlang der Buchsen- oder Lagerschalenkontur, wobei entlang der Nuten ein Steg aus Restmaterial als Sollbruchstelle erzeugt wird,
- Rollen oder Biegen des Flachbandmaterials auf Buchsen- oder Lagerschalenform,
- ein Auslagern bei einer Temperatur zwischen 300°C bis 450°C und einer Prozesszeit zwischen 1 h bis 19 h,
- mechanisches Trennen des entlang der Nuten verlaufenden Stegmaterials,
- Erhalt einer fertigen Gleitlagerbuchse oder Lagerschale, die ihre endgültige Form aufweist.

2. Verfahren nach Anspruch 1, **dadurch gekennzeichnet, dass** die Nuten (3) mittels Prägen eingebracht werden.

3. Verfahren nach Anspruch 1, **dadurch gekennzeichnet, dass** die Nuten (3) mittels Fräsen eingebracht werden.

4. Verfahren nach Anspruch 1, **dadurch gekennzeichnet, dass** die Nuten (3) mittels Laserkerben eingebracht werden.

5. Verfahren nach Anspruch 1, **dadurch gekennzeichnet, dass** die Nuten (3) mittels Rollkerben eingebracht werden.

6. Verfahren nach einem der Ansprüche 1 bis 5, **dadurch gekennzeichnet, dass** als Kupferlegierung eine Legierung auf der Basis von Cu-Sn-Ni verwendet wird.

7. Verfahren nach einem der Ansprüche 1 bis 6, **dadurch gekennzeichnet, dass** nach dem Abtrennen entlang der Nuten (3) die durch die Nuten vorgegebenen Seitenflächen entgratet werden.

8. Verfahren nach einem der Ansprüche 1 bis 7, **dadurch gekennzeichnet, dass** das Gleitlager (2) beschichtet wird.

9. Gerollte oder gebogene Gleitlagerbuchse oder Lagerschale, erhalten durch das Verfahren nach einem der Ansprüche 1 bis 8.

## Claims

1. Method for producing a plain bearing sleeve or bearing shell (2) having a variable width from a flat strip material, comprising a curable copper alloy, **characterised by** the following steps:
- introducing grooves (3) in the flat strip material along the sleeve or bearing shell contour, a web of residual material being produced as a predetermined breaking point along the grooves,
- rolling or bending the flat strip material into sleeve or bearing shell form,
- age-hardening at a temperature of from 300°C to 450°C and a process time of between 1 hour and 19 hours,
- mechanically separating the web material which extends along the grooves,
- obtaining a finished plain bearing sleeve or bearing shell having its final shape.

2. Method according to claim 1, **characterised in that** the grooves (3) are formed by means of stamping.

3. Method according to claim 1, **characterised in that** the grooves (3) are formed by means of milling.

4. Method according to claim 1, **characterised in that** the grooves (3) are formed by means of laser notching.

5. Method according to claim 1, **characterised in that** the grooves (3) are formed by means of roll notching.

6. Method according to any one of claims 1 to 5,
**characterised in that** an alloy based on Cu-Sn-Ni is used as a copper alloy.

7. Method according to any one of claims 1 to 6,
**characterised in that**, after separation along the grooves (3), the side faces predetermined by the grooves are deburred.

8. Method according to any one of claims 1 to 7,
**characterised in that** the plain bearing (2) is coated.

9. Rolled or bent plain bearing sleeve or bearing shell, obtained by the method according to any one of claims 1 to 8.

## Revendications

1. Procédé de fabrication d'une douille de palier lisse ou d'un coussinet de palier (2) de largeur variable, à partir d'un matériau plat en bande, constitué d'un alliage de cuivre pouvant être durci, **caractérisé par** les étapes suivantes :
- réalisation de rainures (3) dans le matériau plat en bande le long du contour de douille ou de coussinet de palier, une nervure de matériau résiduel étant engendrée le long des rainures, en guise de zone de rupture programmée,
- roulage ou cintrage du matériau plat en bande à la forme de douille ou de coussinet de palier,
- traitement de précipitation à une température entre 300°C à 450°C et pendant une durée de processus comprise entre 1 h et 19 h,
- sectionnement mécanique du matériau de nervure s'étendant le long des rainures,
- obtention d'une douille de palier lisse ou d'un coussinet de palier fini, qui présente sa forme définitive.

2. Procédé selon la revendication 1, **caractérisé en ce que** les rainures (3) sont réalisées par estampage.

3. Procédé selon la revendication 1, **caractérisé en ce que** les rainures (3) sont réalisées par fraisage.

4. Procédé selon la revendication 1, **caractérisé en ce que** les rainures (3) sont réalisées par entaillage au laser.

5. Procédé selon la revendication 1, **caractérisé en ce que** les rainures (3) sont réalisées par entaillage à la molette.

6. Procédé selon l'une des revendications 1 à 5, **caractérisé en ce qu'**en guise d'alliage de cuivre on utilise un alliage à base de Cu-Sn-Ni.

7. Procédé selon l'une des revendications 1 à 6, **caractérisé en ce qu'**après le sectionnement le long des rainures (3), les surfaces latérales prédéfinies par les rainures, sont ébarbées.

8. Procédé selon l'une des revendications 1 à 7, **caractérisé en ce que** l'on réalise un revêtement sur le palier lisse (2).

9. Douille de palier lisse ou coussinet de palier formé par roulage ou cintrage, obtenu par le procédé selon l'une des revendications 1 à 8.
